# FASCICULE DE BREVET EUROPEEN

(11) **EP 2 043 985 B1**
(45) Date de publication et mention de la délivrance du brevet: **26.07.2017**
(21) Numéro de dépôt: 07803788.4
(22) Date de dépôt: 27.06.2007
(51) Int. Cl.: C07C 37/68, C07C 37/74, C07C 37/84, C07C 39/08

(54) **PROCÉDÉ DE PRÉPARATION D'HYDROQUINONE PURIFIÉE ET MISE EN FORME**
VERFAHREN ZUR HERSTELLUNG VON GEREINIGTEM HYDROCHINON UND DESSEN ZUBEREITUNG
METHOD FOR PREPARING PURIFIED HYDROQUINONE AND FORMING SAME

(30) Priorité: 29.06.2006 FR 0605865
(43) Date de publication de la demande: 08.04.2009
(73) Titulaire: RHODIA OPERATIONS, 75009 Paris (FR)
(72) Inventeur: GAYET, Hubert, F-69800 Saint-Priest (FR); HEINISCH, Bruno, F-69100 Villeurbanne (FR); LE THIESSE, Jean-Claude, F-42100 Saint-Etienne (FR)
(74) Mandataire: Menville, Laure
(86) Numéro de dépôt international: PCT/FR2007/001079
(87) Numéro de publication internationale: WO 2008/000955

(56) Documents cités:
- EP-A- 0 740 954
- FR-A1- 2 788 763
- FR-A1- 2 846 324
- GB-A- 2 083 816
- JP-A- 2000 302 716

## Description

La présente invention a pour objet un procédé de préparation d'hydroquinone purifiée et mise en forme.

L'invention vise à fournir l'hydroquinone débarrassée des impuretés résultantes de son procédé de préparation et mise en forme.

Selon un mode de réalisation, l'invention fournit un procédé permettant d'obtenir de l'hydroquinone de grande pureté et mise en forme.

L'hydroquinone (ou 1,4-dihydroxybenzène) est un produit largement utilisé dans de nombreux domaines d'application en tant qu'inhibiteur de polymérisation, anti-oxydant dans les élastomères ou comme intermédiaire de synthèse. Un autre domaine d'application est la photographie. Il s'ensuit que c'est un produit de grande consommation.

Une pureté différente est requise selon les marchés concernés.

En effet, si certaines applications, se satisfont d'une hydroquinone technique c'est-à-dire renfermant quelques impuretés afin de minimiser les coûts d'exploitation, d'autres applications en particulier la photographie requièrent un degré de pureté très élevée.

Ainsi, l'hydroquinone doit satisfaire dans certains cas à des exigences de pureté élevée qui peuvent être assez contraignantes. FR2788763 divulgue un procédé de purification de l'hydroquinone comprenant des étapes de distillation et de raffinage à partir d'un mélange hydroquinone-résorcinol-catéchol. Le problème qui se pose est que la purification n'est pas aisée car l'hydroquinone est un produit sensible à l'oxydation et qui conduit vite à des produits de dégradation qui sont colorés.

Dans l'hydroquinone brute à purifier selon l'invention, il y a essentiellement de l'hydroquinone c'est-à-dire au moins 90 % en masse d'hydroquinone et le reste étant constitué par les impuretés à éliminer. Préférentiellement, l'hydroquinone brute comprend au moins 96 % en masse d'hydroquinone.

Or, il y a une difficulté grande à éliminer des teneurs extrêmement faibles d'impuretés.

La difficulté est par ailleurs accrue de par la nature des impuretés. Les composés à séparer ont des volatilités proches car il y a présence d'isomères de l'hydroquinone et il y a également présence de pyrogallol parmi les impuretés à éliminer.

Or, la séparation du pyrogallol présent dans le mélange des impuretés à éliminer, pose problème à l'Homme du Métier. En effet, le pyrogallol est un composé qui se dégrade thermiquement encore plus facilement que l'hydroquinone et sa décomposition conduit à des impuretés colorées.

Par ailleurs, des caractéristiques physico-chimiques différentes, en termes de granulométrie, coulabilité, vitesse de dissolution peuvent être requises selon les applications.

L'hydroquinone est actuellement disponible sur le marché sous la forme d'une poudre formée d'aiguilles petites et cassantes. Les inconvénients qui en résultent sont la présence de fines qui entraînent des problèmes de poussiérage lors du stockage et de la manipulation de ladite poudre.

Or les poussières d'hydroquinone ne sont pas sans danger par rapport à l'environnement en raison de risques d'explosion et eu égard à l'Homme car cette substance est irritante pour les yeux, les voies respiratoires et peut également provoquer des irritations de la peau lorsqu'elle est mise à son contact.

Donc, le marché est demandeur d'hydroquinone présentant une pureté élevée mais ne présentant pas les problèmes liés à la manipulation d'une poudre.

Des procédés alternatifs de mise en forme sont décrit dans l'art antérieur. Par exemple, la demande de brevet EP 0740954 décrit a procédé de prodution de produits chimique sous forme de fines particules solides. La demande de brevet JP 2000-302716 décrit un procédé de préparation de phénols sous forme granulaire à partir de phénols en poudre. Enfin, la demande de brevet FR 2 846 324 décrit un procédé de préparation de perles d'hydroquinone.

Une des voies de synthèse de l'hydroquinone consiste à effectuer l'hydroxylation du phénol par le peroxyde d'hydrogène, notamment en présence de catalyseurs acides homogènes ou hétérogènes.

Ainsi, on peut faire appel comme selon FR 2 071 464, a un acide protique fort c'est-à-dire un acide présentant un pKa dans l'eau inférieur à 0,1, de préférence inférieur à -1.

Comme exemples d'acides protiques forts, on peut mentionner entre autres, l'acide sulfurique, l'acide chlorosulfurique, l'acide perchlorique, les acides sulfoniques comme par exemple, l'acide méthanesulfonique, trifluorométhanesulfonique, toluènesulfonique, phénolsulfonique.

Comme autres exemples de catalyseurs acides protiques, on peut citer les résines sulfoniques et plus particulièrement les résines commercialisées sous différentes dénominations commerciales. On peut citer, entre autres, les résines suivantes : TEMEX 50, AMBERLYST 15, AMBERLYST 35, AMBERLYST 36, DOWEX 50W.

Les résines précitées sont constituées d'un squelette polystyrénique qui porte des groupes fonctionnels qui sont des groupes sulfoniques. Le squelette polystyrénique est obtenu par polymérisation du styrène et du divinylbenzène, sous l'influence d'un catalyseur d'activation, le plus souvent un peroxyde organique, ce qui conduit à un polystyrène réticulé qui est ensuite traité par de l'acide sulfurique ou sulfochlorique concentré conduisant à un copolymère styrène-divinylbenzène sulfoné.

Il est également possible de faire appel à des résines sulfoniques qui sont des copolymères phénol-formol et qui portent sur le noyau aromatique un groupe méthylènesulfonique, par exemple la résine commercialisée sous la dénomination DUOLITE ARC 9359.

D'autres résines disponibles sur le marché conviennent également et l'on peut citer les résines perfluorées porteuses de groupes sulfoniques et, plus particulièrement le NAFION qui est un copolymère de tétrafluoroéthylène et de perfluoro[2-(fluorosulfonyléthoxy)-propyl] vinyl éther.

Comme autres catalyseurs convenant dans les procédés d'hydroxylation, on peut mentionner les complexes de fer II et de cuivre II (FR 2 121 000, USSR 1 502 559) et tout autre catalyseur de type Fenton.

D'autres procédés de préparation de l'hydroquinone font appel à une catalyse hétérogène. Ainsi, on peut mettre en oeuvre une zéolithe acide de type silicalite de titane (ou titanosilicalite-1) ou de fer de type TS-1 (FR 2 489 816), une zéolithe de type silicalite de titane MEL (EP 1 131 264) ou une titanozéosilite de type MFI (EP 1 123 159). Il est également possible d'utiliser une zéolithe MCM-22 (FR 2 856 681).

A l'issue de telles réactions d'hydroxylation, on obtient un mélange comprenant essentiellement du pyrocatéchol (ou 1,2-dihydroxybenzène) et de l'hydroquinone, en proportions variables avec en général, un ratio massique pyrocatéchol/ hydroquinone de l'ordre de 0,25 à 4,0, ainsi que différents sous-produits en des quantités beaucoup plus faibles, notamment du résorcinol (ou 1,3-dihydroxybenzène) et du pyrogallol (ou 1,2,3-trihydroxybenzène), généralement à des teneurs de l'ordre de 0,5 à 4,0 % en masse, pourcentages exprimés par rapport à la quantité d'hydroquinone et de pyrocatéchol formée.

On obtient des mélanges de compositions variables comprenant, en masse, de 20 à 80% de pyrocatéchol, de 80 à 20 % d'hydroquinone, de 0,1 à 2 % de résorcinol et de 0,1 à 2 % de pyrogallol.

Typiquement, on obtient des mélanges comprenant, en masse, de 50 à 80 % de pyrocatéchol, de 20 à 50 % d'hydroquinone, de 0,1 à 2 % de résorcinol et de 0,1 à 2 % de pyrogallol.

Pour isoler l'hydroquinone à partir de mélanges bruts de ce type, une méthode actuellement connue consiste à effectuer la distillation dudit mélange permettant d'obtenir en tête de distillation le pyrocatéchol (qui est le composé le plus volatil du mélange), et en pied de distillation, une "hydroquinone brute", à savoir un mélange comprenant essentiellement de l'hydroquinone, associée à de faibles quantités d'impuretés (notamment résorcinol et pyrogallol ainsi que d'éventuelles traces de pyrocatéchol non éliminées par la distillation).

L'invention propose à partir de cette hydroquinone brute, de fournir de l'hydroquinone pouvant avoir une très haute pureté et étant présentée sous une forme permettant d'éviter les problèmes liés à la manipulation d'une poudre.

Il a maintenant été trouvé et c'est ce qui constitue l'objet de la présente invention, un procédé de préparation d'hydroquinone purifiée et mise en forme sous forme d'écailles dont le pourcentage en masse de particules de dimension inférieure à 100 µm est inférieur à 3%, à partir d'une hydroquinone brute *H*Q*⁰* contenant de 96 à 99,5 % en masse d'hydroquinone, et de 0,5 et 4 % en masse d'impuretés, lesdites d'impuretés incluant au moins du résorcinol, du pyrogallol et des traces de pyrocatéchol caractérisé par le fait qu'il comprend les étapes suivantes :
- une étape de purification de l'hydroquinone brute par distillation comprenant :
   (A) une distillation d'étêtage, dans laquelle on injecte l'hydroquinone brute *HQ*⁰ dans une colonne de distillation et où on élimine le résorcinol en tête de distillation éventuellement conjointement à tout ou partie des autres impuretés légères ayant une température de vaporisation inférieure à celle de l'hydroquinone, ce par quoi on récupère en pied de colonne un mélange brut M comprenant de l'hydroquinone et les impuretés lourdes ; et
   (B) une distillation d'équeutage dans laquelle on injecte le mélange brut M obtenu dans l'étape (A) dans une colonne de distillation et où on élimine le pyrogallol en pied de colonne éventuellement conjointement à tout ou partie des autres impuretés lourdes ayant une température de vaporisation supérieure à celle de l'hydroquinone, ce par quoi on récupère en tête de colonne de l'hydroquinone sous une forme purifiée *(HQ) ;*
   les étapes de distillation (A) et (B) étant conduites sous atmosphère inerte et de telle sorte que la température dans les colonnes de distillation soit d'au moins 175°C, la pression soit comprise entre 50 et 100 millibars et le temps de séjour inférieur à 1 heure,
- une étape de mise en forme de l'hydroquinone purifiée obtenue en sortie de distillation, par dépôt de celle-ci, à l'état liquide, en film sur un support en un matériau ou revêtu d'un matériau conducteur de la chaleur suivie par sa solidification en amenant le support à la température adéquate, puis récupération de l'hydroquinone solidifiée sous forme d'écailles, à l'aide de tout moyen approprié.

Ainsi, l'invention propose un procédé qui enchaîne une étape de purification par distillation suivie immédiatement par sa mise en forme. Il en résulte que l'hydroquinone n'est pas soumise à une opération de fusion additionnelle généralement requise pour la mise en forme. Le procédé de l'invention qui met en oeuvre l'hydroquinone issue directement de la distillation, évite des dégradations éventuelles et des possibles colorations subséquentes.

Le procédé proposé par l'invention est simple et moins coûteux en investissement par rapport à d'autres techniques de mise en forme comme le prilling décrit dans EP-A 1556322.

L'hydroquinone obtenue présente une pureté élevée et est aisément manipulable.

Conformément au procédé de l'invention, on effectue dans une première étape du procédé de l'invention, la purification de l'hydroquinone brute.

Selon la première étape du procédé de l'invention, il est possible d'effectuer la purification d'une hydroquinone brute (HQ°) contenant essentiellement de l'hydroquinone à raison d'au moins 90 % en masse et de faibles quantités d'impuretés inférieures à 10 % en masse, où les impuretés présentes incluent à la fois :
(i) des impuretés ayant une température de vaporisation inférieure à celle de l'hydroquinone, dites ci-après "*impuretés légères",* incluant en particulier du résorcinol, de préférence à titre d'impureté majoritaire parmi les impuretés légères ; et
(ii), des impuretés ayant une température de vaporisation supérieure à celle de l'hydroquinone, désignées ci-après par *"impuretés lourdes",* incluant en particulier du pyrogallol, de préférence à titre d'impureté majoritaire parmi les impuretés lourdes.

Conformément à l'invention, l'étape de purification du procédé de l'invention comprend une distillation d'étêtage (A) et une distillation d'équeutage (B).

Les inventeurs ont trouvé que la mise en oeuvre des étapes successives de distillation (A) et (B) ci-dessus permet d'obtenir une purification efficace de l'hydroquinone, avec la possibilité d'éliminer des quantités extrêmement faibles d'impuretés comprenant un mélange de composés plus volatiles que l'hydroquinone et de composés moins volatiles que l'hydroquinone, de type résorcinol et pyrogallol.

Dans ce cadre, il est à noter que le procédé de l'invention peut être utilisé pour traiter des hydroquinones brutes contenant de l'ordre de 0,5 à 4 % en masse d'impuretés, par exemple des quantités d'impuretés aussi faibles que de 0,5 à 2 % en masse, pour conduire *in fine* à des hydroquinones purifiées contenant en général moins de 4000 ppm d'impuretés, le plus souvent moins de 3000 ppm.

De façon plus spécifique, la purification selon l'invention peut notamment être mise en oeuvre pour la préparation d'hydroquinones de haute pureté, contenant des impuretés à une teneur de moins de 2500 ppm, typiquement au plus de l'ordre de 2000 ppm, de préférence au plus de l'ordre de 1500 ppm, et plus préférentiellement au plus de l'ordre de 1000 ppm, voire moins.

La possibilité d'une telle efficacité de séparation s'avère relativement inattendue, dans la mesure où le problème qui se posait en termes de séparation était particulièrement difficile à résoudre, notamment compte tenu du fait que les différents composés à séparer ont des volatilités relatives très proches.

En outre l'hydroquinone présente une haute température de fusion (172,5°C) ainsi qu'une température de vaporisation très élevée, même sous pression réduite (258°C sous 500 millibars ; 208°C sous 100 millibars).

De façon encore plus inattendue, il s'avère que, bien que l'hydroquinone soit sensible à la dégradation thermique et qu'il soit nécessaire de maintenir l'hydroquinone à des températures de 170 à 220°C tout au long de la distillation des étapes (A) et (B), ces étapes peuvent néanmoins être conduites de façon efficace tout en limitant les phénomènes de dégradation thermique de l'hydroquinone, qui sont susceptibles de former des produits de dégradation colorés de type quinones.

Dans ce cadre, les inventeurs ont en particulier mis en évidence que les étapes (A) et (B) peuvent être conduites efficacement en limitant néanmoins le temps de séjour dans les colonnes de distillation, ce par quoi les phénomènes de dégradation thermique peuvent être très sensiblement inhibés.

Ces phénomènes de dégradation peuvent en outre encore être davantage évités en limitant la présence d'oxygène dans les colonnes de distillation, par exemple en travaillant sous atmosphère inerte.

En outre, les travaux des inventeurs ont permis d'établir que, dans les conditions de purification selon l'invention, dans le cas où d'éventuels produits de dégradation thermiques colorés de l'hydroquinone de type quinones se forment, ceux-ci sont substantiellement récupérés avec le pyrogallol en pied de colonne de la distillation d'équeutage de l'étape (B).

Selon la présente invention, il a également été découvert que le pyrogallol et les impuretés résultant de sa dégradation pouvaient être éliminées d'une manière efficace au cours d'une opération de distillation.

Comme mentionné précédemment, le pyrogallol se décompose au moins partiellement en impuretés colorées du fait que la distillation de l'hydroquinone est effectuée à température élévée.

Il était à craindre que les impuretés colorées se retrouvent, dans l'étape (B) en tête de distillation avec l'hydroquinone.

Il a été trouvé selon l'invention que le pyrogallol ainsi que les impuretés formées par sa dégradation restaient en pied de distillation et pouvaient donc être efficacement séparées de l'hydroquinone.

L'invention fournit un procédé de purification permettant une élimination à la fois efficace et économique des différentes impuretés.

La composition exacte de l'hydroquinone brute *HQ⁰* traitée selon les étapes (A) et (B) du procédé de l'invention peut varier en une assez large mesure, le procédé de l'invention se révélant toutefois surtout intéressant pour des hydroquinones brutes contenant de 96 à 99,5 % en masse d'hydroquinone et des teneurs en impuretés de l'ordre de 0,5 à 4 %, par exemple de 0,5 à 2 % en masse, notamment de 1 à 2 % en masse par rapport à la masse totale de l'hydroquinone brute.

Typiquement, une hydroquinone brute *HQ⁰* traitée selon l'invention contient de 0,1 à 2 %, par exemple de 0,2 à 1 % en masse, d'impuretés légères (ayant une température de vaporisation inférieure à celle de l'hydroquinone), incluant du résorcinol. Le résorcinol est en général une impureté majoritaire au sein des impuretés légères, les impuretés légères comprenant en règle générale au moins 50 % en masse de résorcinol par rapport à la masse totale des impuretés légères, par exemple au moins 70 %, notamment au moins 80 %, en particulier au moins 90 % en masse, voire plus. Outre le résorcinol, les impuretés légères présentes dans l'hydroquinone brute *HQ⁰* peuvent notamment comprendre du pyrocatéchol.

Par ailleurs, dans l'hydroquinone brute *HQ⁰*, la quantité d'impuretés lourdes (ayant une température de vaporisation supérieure à celle de l'hydroquinone) est usuellement de 0,1 à 2 %, par exemple de 0,2 à 1 % en masse. Ces impuretés lourdes incluent en particulier du pyrogallol, généralement à titre d'impureté lourde majoritaire, en général en association avec d'autres impuretés lourdes, notamment des goudrons ou bien encore des produits de dégradation thermique de l'hydroquinone tels que des quinones. Ainsi, les impuretés lourdes comprennent en général au moins 50 % en masse de pyrogallol par rapport à la masse totale des impuretés lourdes, par exemple au moins 70 %, voire au moins 80 %, en particulier au moins 90 % en masse ou plus.

Selon un mode de réalisation particulier, l'hydroquinone brute *HQ^{o}* traitée selon les étapes (A) et (B) est obtenue ou susceptible d'être obtenue, à partir d'un mélange réactionnel issu d'une hydroxylation de phénol par le peroxyde d'hydrogène en présence de catalyseurs acides du type cité plus haut dans la présente description, après élimination substantielle du pyrocatéchol par distillation.

Une hydroquinone brute *HQ⁰* adaptée au procédé de l'invention comprend, en masse par rapport à la quantité totale d'hydroquinone brute :
- de 96 à 99,5 % d'hydroquinone,
- de 0,1 à 2 %, de préférence de 0,2 à 1 % de résorcinol,
- de 0,1 à 2 %, de préférence de 0,2 à 1 % de pyrogallol
- éventuellement du pyrocatéchol sous forme de traces, typiquement à une teneur inférieure à 500 ppm (0,05 %), de préférence à une teneur inférieure à 100 ppm (0,01 %).

Quelle que soit la nature exacte de l'hydroquinone brute *HQ⁰* traitée selon le procédé de l'invention, les étapes de distillation (A) et (B) sont avantageusement mises en oeuvre dans les conditions exposées ci-après.

L'étape (A) de distillation d'étêtage vise à éliminer le résorcinol et de préférence, sensiblement toutes les impuretés légères présentes dans l'hydroquinone brute *HQ⁰,* en les entraînant en tête de colonne, pour récupérer en pied de colonne un mélange brut M comprenant essentiellement de l'hydroquinone et des impuretés lourdes, appauvri en impuretés légères, en particulier en résorcinol.

Il est à noter que l'élimination des impuretés en tête de colonne s'accompagne en général d'un départ d'une fraction d'hydroquinone en tête de colonne, qui n'est donc pas récupérée dans le mélange brut M qui sera mis en oeuvre dans l'étape (B). Pour limiter cette perte d'hydroquinone dans l'étape (A), on peut notamment jouer sur le nombre d'étages théoriques et le taux de reflux de la colonne de distillation utilisée dans l'étape (A), ce par quoi on peut typiquement obtenir dans le procédé un ratio (hydroquinone perdue/hydroquinone dans le mélange M) inférieur à 2 %, par exemple compris entre 0,2 et 1 %, notamment entre 0,3 et 0,7 %.

Le débit d'alimentation de l'hydroquinone brute *HQ⁰* dans la colonne de distillation de l'étape (A) peut varier en une assez large mesure, notamment en fonction du dimensionnement choisi pour la colonne et du débit recherché *in fine* pour l'hydroquinone purifiée. A titre non limitatif, on peut simplement préciser qu'il est possible de travailler avec des débits d'alimentation allant jusqu'à 3000 kg/ kg/h, voire jusqu'à 5000 kg/h. Typiquement, on peut employer des débits de l'ordre de 100 à 3000 kg/h.

Dans l'étape (A), le point d'alimentation où est introduit l'hydroquinone brute *HQ⁰* est en général sensiblement à mi-hauteur de la colonne de distillation, à savoir avec un rapport volumique de la zone de rectification de la colonne de l'étape (A) à la zone d'épuisement de la colonne de l'étape (A) en général compris entre 25 : 75 et 75 : 25, plus préférentiellement entre 30 : 70 et 70 : 30, par exemple entre 40 : 60 et 60 : 40. On entend ici par "zone de rectification" le volume interne de la colonne de distillation de l'étape (A) qui est situé au-dessus du plan horizontal contenant le point d'alimentation, par opposition à la "zone d'épuisement", correspondant au volume interne de colonne situé en dessous de ce plan horizontal.

Le flux qui sort en tête de la colonne de distillation de l'étape (A), qui contient essentiellement les impuretés légères à éliminer et un peu d'hydroquinone, peut avantageusement être dérivé en partie, pour être réinjecté dans la colonne de distillation, selon la technique du reflux. La quantité de flux qui est réinjecté dans la colonne peut être quantifiée par un taux de reflux, défini par le rapport du débit sortant effectivement à la sortie de la tête de colonne, rapporté au débit de matière réinjecté de la tête de la colonne vers l'intérieur de la colonne. Dans la colonne de la distillation d'étêtage de l'étape (A), ce taux de reflux est avantageusement compris entre 300 et 2000, typiquement entre 400 et 1500, par exemple entre 500 et 1000.

Par ailleurs, le nombre d'étages théoriques de la colonne utilisée dans l'étape (A) est avantageusement d'au moins 20, de préférence d'au moins 30, par exemple entre 30 et 50.

D'autre part, le temps de séjour de l'hydroquinone dans la colonne de l'étape (A) est inférieur à 1 heure, de préférence inférieur à 45 minutes, et plus préférentiellement encore inférieur à 30 minutes, ce qui permet notamment d'inhiber les phénomènes de dégradation thermique de l'hydroquinone, en limitant le temps pendant lequel celle-ci est soumise à haute température. Néanmoins, ce temps de séjour reste en général au moins égal à 10 minutes, par exemple d'au moins 15 minutes notamment pour permettre une séparation efficace des impuretés légères dans l'étape (A). On obtient ainsi un bon compromis dans l'étape (A) entre séparation et inhibition de la dégradation thermique avec des temps de séjour typiquement de l'ordre de 15 à 30 minutes.

L'étape (B) qui suit l'étape (A) consiste quant à elle, en une distillation d'équeutage, qui vise à éliminer le pyrogallol présent dans le mélange brut M obtenu à l'issue de la distillation d'étêtage, et de préférence sensiblement toutes les impuretés lourdes. Dans l'étape (B), à l'inverse de l'étape (A), ce sont les impuretés qui sont envoyées en pied de colonne, et c'est en tête de colonne que l'on récupère l'hydroquinone sous forme purifiée.

Là également, l'élimination des impuretés vers le pied de colonne s'accompagne d'un départ d'une partie de l'hydroquinone en pied de colonne.

Ainsi, on ne récupère pas la totalité de l'hydroquinone du mélange M en tête de colonne. Pour limiter cette perte d'hydroquinone en pied de colonne de distillation de l'étape (B), on peut notamment jouer sur le taux de reflux et sur le nombre d'étages théoriques de la colonne de distillation de l'étape (B), ce par quoi on peut typiquement obtenir dans l'étape (B) un ratio (hydroquinone perdue/hydroquinone récupérée dans l'hydroquinone purifiée) inférieur à 2 %, par exemple compris entre 0,2 et 1 %.

Dans l'étape (B), le point d'alimentation où est introduit le mélange brut M issu du pied de colonne de l'étape (A) est en général sensiblement à mi-hauteur de la colonne de distillation. Typiquement, pour la colonne de l'étape (B), le rapport volumique de la zone de rectification à la zone d'épuisement est compris entre 25:75 et 75:25, plus préférentiellement entre 30:70 et 70 : 30, par exemple entre 40 : 60 et 60 : 40. Là encore, on entend par "zone de rectification" le volume interne de la colonne de distillation de l'étape (B) qui est situé au-dessus du plan horizontal contenant le point d'alimentation, par opposition à la "zone d'épuisement", correspondant au volume interne de la colonne situé en dessous de ce plan horizontal.

Par ailleurs, comme dans l'étape (A), on travaille avantageusement à reflux dans l'étape (B), à savoir en dérivant une partie du flux qui sort en tête de la colonne de distillation de l'étape (B), qui contient de l'hydroquinone purifiée, pour le réinjecter dans la colonne de distillation. Le taux de reflux dans la colonne de la distillation d'équeutage de l'étape (B), défini par le rapport du débit sortant effectivement à la sortie de la tête de colonne, rapporté au débit de matière réinjectée de la tête de la colonne vers la colonne, est avantageusement compris entre 1 et 15, typiquement entre 3 et 12, par exemple entre 4 et 10.

Par ailleurs, le nombre d'étages théoriques de la colonne utilisée dans l'étape (B) est avantageusement au moins égal à 20, de préférence au moins égal à 30, par exemple entre 30 et 50.

Dans l'étape (B), il est tout particulièrement important de contrôler le temps de séjour de l'hydroquinone dans la colonne de distillation.
D'une part, pour éviter la dégradation thermique du produit, on choisit un temps de séjour inférieur à 1 heure, plus avantageusement inférieur à 30 minutes dans la colonne de l'étape (B). Pour obtenir une séparation efficace des impuretés lourdes dans l'étape (B), il est toutefois préférable, le plus souvent, de travailler avec des temps de séjour de l'hydroquinone dans la colonne de l'étape (B) d'au moins 10 minutes, par exemple entre 15 et 30 minutes.

De façon plus générale, il est à noter que les étapes (A) et (B) sont conduites dans des conditions permettant la distillation de l'hydroquinone, ce qui implique notamment qu'elles sont conduites à des températures suffisantes pour que l'hydroquinone se présente à l'état liquide ou gazeux. Il est indiqué d'éviter la présence de tout point froid en deçà de 170°C (température de solidification de l'hydroquinone) dans le dispositif de mise en oeuvre des étapes de distillations (A) et (B), ce qui pourrait induire des phénomènes d'encrassement des colonnes, nuisibles au rendement et/ou à la qualité de la distillation, voire des phénomènes de prise en masse, qui nécessiteraient un arrêt complet du procédé et des opérations onéreuses de nettoyage de l'installation. A cet effet, par sécurité, toutes les zones internes des colonnes de distillation mises en oeuvre pour les étapes (A) et (B) sont au minimum à une température de 175°C, et de préférence à une température d'au moins 180°C, par exemple d'au moins 185°C. La plupart des zones sont supérieures à ces températures, pour parvenir à la vaporisation de l'hydroquinone nécessaire à la distillation, la température restant néanmoins typiquement inférieure à 220°C.

En règle générale, pour obtenir les hautes températures requises, sans présence de points froids, on met avantageusement en oeuvre des colonnes à double enveloppe, en faisant circuler un fluide caloporteur porté à une température de l'ordre de 180 à 220°C. Comme fluides caloporteurs appropriés, on peut notamment citer les esters lourds d'acides carboxyliques, comme le phtalate d'octyle, les éthers aromatiques tels que l'oxyde de biphényle et/ou l'oxyde de benzyle, le diphényle, les terphényles, les autres polyphényles éventuellement partiellement hydrogénés, les huiles paraffiniques et/ou naphténiques, ou bien encore certains résidus de distillation du pétrole.

Il est par ailleurs souhaitable d'éviter au maximum d'établir des ponts thermiques entre le dispositif mis en oeuvre selon l'invention et le milieu extérieur, pour se prémunir de tout risque de déperditions thermiques.

Par ailleurs, compte tenu des hautes températures utilisées, il est le plus souvent souhaitable d'éviter la présence d'oxygène dans les étapes de distillation des étapes (A) et (B), notamment pour éviter une dégradation de l'hydroquinone en quinones. A cet effet, ces étapes sont conduites sous une atmosphère inerte, par exemple sous azote ou bien encore sous argon, l'azote étant préféré notamment compte tenu de son coût réduit.

D'autre part, notamment pour éviter d'avoir à chauffer à des températures trop importantes, les distillations de chacune des étapes (A) et (B) sont avantageusement conduites sous pression réduite, ces pressions, identiques ou différentes dans les colonnes de distillation des étapes (A) et (B) sont comprises entre 50 et 100 millibars, par exemple entre 60 et 90 millibars. Les pressions de travail des étapes (A) et (B) peuvent être identiques ou différentes.

La distillation des étapes (A) et (B) peut avantageusement être conduite selon un mode continu, notamment en injectant à débit constant l'hydroquinone brute *HQ⁰* à traiter à l'entrée de la colonne de distillation d'étêtage. Il n'est toutefois pas exclu de la conduire selon un mode discontinu.

Quel que soit leur mode exact de mise en oeuvre, les étapes (A) et (B) conduisent *in fine,* en tête de la colonne de l'étape (B), à une hydroquinone purifiée *HQ* après condensation, obtenue à l'état liquide.

L'hydroquinone purifiée obtenue selon le procédé de l'invention comprend un taux très réduit d'impuretés, en général moins de 4000 ppm d'impuretés, le plus souvent moins de 3000 ppm.

Selon un mode de réalisation particulier, le procédé de l'invention peut être mis en oeuvre pour la préparation d'hydroquinone de haute pureté, contenant typiquement moins de 2500 ppm, préférentiellement moins de 2000 ppm d'impuretés.

Une telle hydroquinone de haute pureté contient avantageusement moins de 2000 ppm d'impuretés légères telles que du résorcinol ou du pyrocatéchol (traces), cette teneur en impuretés légères étant de préférence inférieure à 1500 ppm, par exemple entre 1000 et 1500 ppm et plus préférentiellement entre 300 et 1000 ppm. La teneur en impuretés lourdes est quant à elle avantageusement inférieure à 500 ppm, de préférence inférieure à 300 ppm, par exemple entre 20 et 200 ppm.

Cette étape du procédé de l'invention permet d'éliminer efficacement les impuretés puisque les teneurs de résorcinol et de pyrogallol peuvent descendre respectivement jusqu'à 300 ppm et 20 ppm et le pyrocatéchol n'est plus détectable par l'analyse.

Avant la mise en forme, il est souhaitable de disposer d'un bac de stockage dans lequel l'hydroquinone est maintenue à l'état liquide.

Ainsi, ledit bac de stockage est maintenu à une température comprise entre 175°C et 190°C, de préférence comprise entre 178°C et 185°C.

Le chauffage est avantageusement réalisé par circulation de vapeur d'eau ou d'un fluide caloporteur approprié dans la double enveloppe. Comme exemples de fluides caloporteurs, on peut se référer à ceux précédemment cités

Le bac de stockage est maintenu sous atmosphère inerte, de préférence sous atmosphère d'azote.

Conformément au procédé de l'invention, on effectue dans une deuxième étape du procédé de l'invention, la mise en forme de l'hydroquinone purifiée.

De façon plus spécifique, la mise en forme de l'hydroquinone sous forme d'écailles est caractérisée par le fait qu'il comprend les étapes suivantes :
- déposer l'hydroquinone à l'état liquide en film sur un support en un matériau ou revêtu d'un matériau conducteur de la chaleur,
- faire solidifier l'hydroquinone en amenant le support à la température adéquate,
- récupérer le produit solidifié sous forme d'écailles à l'aide de tout moyen approprié.

Selon un mode préféré de réalisation de l'invention, on élimine au préalable l'oxygène de l'enceinte dans laquelle est effectuée l'opération de mise en forme.

Ainsi, la mise en forme de l'hydroquinone à l'état liquide est effectuée dans une atmosphère débarrassée d'oxygène. Selon un mode d'exécution de l'invention, on établit dans l'enceinte une atmosphère de gaz inertes. On peut faire appel à un gaz rare, de préférence l'argon mais généralement on préfère utiliser l'azote en raison de son coût plus faible.

Une fois l'atmosphère inerte établie, on dépose l'hydroquinone à l'état liquide, en film sur un support approprié.

Conformément au procédé de l'invention, l'hydroquinone à l'état liquide est déposée en film sur un support en un matériau ou revêtu d'un matériau conducteur de la chaleur.

Pour le choix du matériau, on fait appel à tout matériau ne réagissant pas avec l'hydroquinone.

Par ailleurs, comme ce matériau a la propriété de conduire la chaleur, on choisit avantageusement un métal ayant une conductibilité thermique d'au moins 10 W/m.K, de préférence entre 15 et 400 W/m.K. Il est à noter que la borne supérieure ne présente aucun caractère critique.

Comme exemples de matériaux répondant aux caractéristiques précitées et convenant tout à fait bien à la mise en oeuvre du procédé de l'invention, on peut mentionner, entre autres, les aciers inoxydables.

On choisit avantageusement les aciers inoxydables, tels que les aciers austénitiques et plus particulièrement les aciers inoxydables 304, 304 L, 316 ou 316 L.

On met en oeuvre un acier ayant au plus 22 % en masse de nickel, le plus souvent compris entre 6 et 20 %, de préférence compris entre 8 et 14 %.

Les aciers 304 et 304 L ont une teneur en nickel variant entre 8 et 12 % et les aciers 316 et 316 L ont une teneur en nickel variant entre 10 et 14 %.

De tels aciers sont utilisés couramment dans l'industrie.

Pour la définition des aciers austénitiques, on peut se référer à l'ouvrage de Robert H. Perry et al, [Perry's Chemical Engineers' Handbook, Sixth Edition (1984), page 23-44].

Le procédé de l'invention est conduit au moyen d'un dispositif permettant la solidification de l'hydroquinone à l'état liquide sur une surface refroidie consistant ou revêtu d'un matériau conducteur qui peut être sous forme d'une bande transporteuse, d'un ou de plateau(x) tournant(s) ou bien d'un cylindre en rotation.

Le dimensionnement de l'appareillage relève de la compétence de l'Homme du Métier.

Les caractéristiques de la bande transporteuse peuvent varier largement. Ainsi, la longueur peut aller par exemple entre 50 cm et 2 m, la largeur entre 1 et 5 m. Pour ce qui est de la vitesse de défilement de la bande, elle peut varier avantageusement entre 1 m/min et 20 m/min.

L'épaisseur des écailles est déterminée en contrôlant la vitesse d'alimentation de l'hydroquinone et la vitesse de défilement de la bande.

Pour ce qui est des plateaux tournants, leur diamètre se situe le plus souvent entre 150 et 400 mm.

Leur vitesse de rotation est choisie préférentiellement entre 5 et 50 tours/min.

Selon une première variante de l'invention, l'hydroquinone à l'état liquide est déposée sur une bande ou sur un ou des plateaux tournants par pulvérisation par l'intermédiaire d'une buse et plus couramment par un système à débordement comprenant une auge d'alimentation remplie en hydroquinone à l'état liquide de manière continue de façon à faire déborder l'hydroquinone qui tombe par gravité sur la bande ou le(s) plateau(x).

L'hydroquinone est solidifiée par refroidissement à une température avantageusement comprise entre 20°C et 80°C.

Le plus souvent, le refroidissement est assuré par pulvérisation d'eau froide sur la face interne de la bande qui n'est pas couverte par l'hydroquinone.

Quant au(x) plateau(x) tournant(s), ils sont généralement constitués d'une double enveloppe dans laquelle circule un liquide de refroidissement, de préférence de l'eau introduite à la température adéquate.

On récupère l'hydroquinone sous forme d'écailles grâce à un couteau racleur.

Selon une autre variante du procédé qui est préférée, l'hydroquinone à l'état liquide est déposée sur un cylindre en rotation.

Le cylindre a des dimensions qui peuvent varier largement.

Ainsi, le diamètre peut aller de 0,15 à 2,5 m, de préférence de 1 à 1,5 m et la longueur peut varier par exemple entre 0,25 à 5 m, de préférence 0,5 à 2 m.

L'alimentation du cylindre peut être faite de multiples manières.

Le cylindre étant placé par exemple dans 1 à 10 cm d'hydroquinone à l'état liquide placée dans une auge d'alimentation, le dépôt sur le cylindre s'effectue par trempage.

Le cylindre tourne et entraîne une couche mince de produit qui se solidifie sur le cylindre par refroidissement.

Le cylindre tourne à une vitesse qui est choisie en fonction de l'épaisseur des écailles souhaitée et la température de l'alimentation.

La couche sera d'autant plus mince que la vitesse de rotation est élevée.

L'alimentation de l'hydroquinone à l'état liquide peut se faire sur le cylindre par l'intermédiaire d'un rouleau applicateur, lui-même alimenté en hydroquinone à l'état liquide.

L'alimentation peut être également faite sur le cylindre par coulée par gravité ou par l'intermédiaire d'une pompe.

Le cylindre est refroidi par circulation d'eau dans une double enveloppe ou par pulvérisation d'eau à l'intérieur du cylindre.

Selon une caractéristique du procédé de l'invention, le cylindre est maintenu de préférence à une température comprise entre 20°C et 80°C et plus préférentiellement entre 30°C et 80°C.

La vitesse de rotation du cylindre varie avantageusement entre 0,5 et 20 tours/min, de préférence entre 3 et 6 tours/min.

L'hydroquinone est maintenue suffisamment longtemps sur le cylindre afin qu'elle se solidifie.

Ensuite, on récupère l'hydroquinone mise en forme à l'aide de tout moyen approprié et plus particulièrement à l'aide d'un couteau qui racle le cylindre et décolle la couche de produit qui est récupéré par tout moyen connu par exemple, par gravité dans un bac de récupération.

Ainsi, un mode de réalisation préféré du procédé de l'invention de préparation des écailles d'hydroquinone comprend les étapes suivantes :
- débarrasser l'oxygène de l'enceinte dans laquelle est effectuée l'opération de mise en forme,
- déposer l'hydroquinone à l'état liquide en film sur un cylindre maintenu à une température comprise entre 20°C et 80°C,
- maintenir l'hydroquinone suffisamment longtemps sur le cylindre afin qu'elle se solidifie.
- récupérer le produit solidifié à l'aide de tout moyen approprié.

On obtient selon le procédé de l'invention de l'hydroquinone de grande pureté et sous forme d'écailles répondant aux caractéristiques donnée ci-après.

Plus précisément, l'hydroquinone est présentée sous l'aspect de particules massives qui ont une forme plaquettaire ; ces particules étant appelées « écailles ».

Les particules plaquettaires répondent à un facteur de forme générale défini en plan par un contour très varié qui peut être plus ou moins carré, rectangulaire, rond ou ovale.

Les différentes écailles de forme variée s'inscrivent dans un parallélépipède présentant les dimensions précisées ci-après.

La longueur varie le plus souvent entre 0,5 et 6 cm, de préférence entre 1 et 3 cm.

La largeur quant à elle, s'échelonne entre 0,5 et 3 cm, de préférence entre 0,5 et 1,5 cm.

Les mesures sont effectuées sur un échantillon de 20 écailles prélevées au hasard.

Les longueur et largeur sont déterminées par mesure à l'aide d'une règle graduée.

Les parallélépipèdes précités possèdent l'une de leurs trois dimensions (l'épaisseur) beaucoup plus faible que les deux autres (largeur et longueur).

Pour ce qui est de l'épaisseur, elle se situe entre 400 µm et 1500 µm, de préférence entre 500 et 750 µm.

L'épaisseur est mesurée à l'aide d'un pied à coulisse ou d'un appareil Palmer.

Il est à noter qu'il n'est pas exclu que certaines particules présentent des dimensions en dehors des limites précédemment données.

La figure 4 représente une photographie prise à l'aide d'un appareil photo numérique qui montre la morphologie de type écaille de l'hydroquinone obtenue selon l'invention.

Il est à souligner que ces particules massives présentent des arrêtes franches.

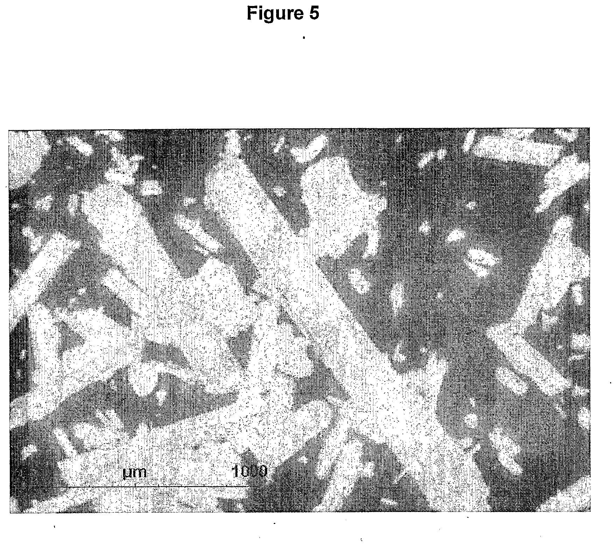

La figure 5 représente une photographie prise à l'aide d'un appareil photo numérique qui montre la morphologie en aiguilles des cristaux de la poudre d'hydroquinone disponible dans le commerce.

La figure 6 représente une photographie prise également à l'aide d'un appareil photo numérique qui montre une meilleure vue d'ensemble du produit de l'invention grâce à un grossissement plus petit et indiqué sur la photographie.

Une caractéristique de l'hydroquinone obtenue est un taux de particules fines très faible par rapport à une présentation sous forme de poudre.

Le taux des particules fines est défini comme le pourcentage en masse des particules de dimensions inférieures à 100 µm.

Sont considérées selon l'invention, comme particules fines, des particules qui passent à travers un tamis ayant une maille de 100 µm.

Le taux des particules fines est inférieur à 3 % en masse, de préférence compris entre 0,7 et 1,5 % en masse, et plus préférentiellement compris entre 0,7 et 1 %.

On précisera à titre indicatif que la taille des particules fines s'échelonnent entre 1 µm et 100 µm, avec un diamètre médian situé entre 20 et 30 µm.

On définit le diamètre médian comme étant tel que 50 % en masse des particules ont un diamètre supérieur ou inférieur au diamètre médian.

A titre de comparaison, on mentionnera que le taux des particules fines de l'hydroquinone sous forme de poudre est de l'ordre de 20 % en masse ce qui signifie que la teneur des particules fines (ou poussières) est divisée par 10 voire même 20.

Afin de définir la granulométrie de l'hydroquinone obtenue, on définit également le pourcentage en masse des particules de dimensions inférieures à 2,5 mm c'est-à-dire des particules qui passent à travers un tamis ayant une maille de 2,5 mm.

Cette teneur se situe généralement entre 20 et 40 % en masse.

A titre de comparaison, on mentionnera que 100 % des particules d'hydroquinone sous forme de poudre sont inférieures à 2,5 mm.

Les écailles d'hydroquinone ont une masse volumique qui peut être plus ou moins élevée. La masse volumique apparente (non tassée) des écailles est de préférence, d'au moins 0,4 g/cm³ et se situe encore préférentiellement entre 0,4 et 0,6 g/cm³, et le plus souvent entre 0,45 et 0,55 g/cm³.

La masse volumique apparente (tassée) des écailles est de préférence, d'au moins 0,5 g/cm³ et se situe encore plus préférentiellement entre 0,5 et 0,8 g/cm³, et le plus souvent entre 0,6 et 0,7 g/cm³.

Les masses volumiques sont mesurées selon le test décrit dans la norme Pharmacopée Européenne [Tome 1, p.256, (2004) 5èmè édition] sur un produit non séché à la seule différence que l'éprouvette de 250 ml est remplacée par une éprouvette de 1 litre.

L'hydroquinone obtenue bien qu'ayant une forme physique lui permettant de résister à l'attrition, conserve une vitesse de dissolution compatible avec une utilisation postérieure.

Ainsi, la vitesse de dissolution des écailles varie selon l'épaisseur desdites écailles.

Le temps de dissolution dans l'eau d'une quantité d'écailles nécessaire pour obtenir une concentration finale d'hydroquinone de la solution de 4,8 % en masse varie entre 10 et 30 min selon l'épaisseur des écailles.

Ces mesures correspondent à un test qui consiste à mesurer le temps nécessaire pour dissoudre ladite quantité dans l'eau maintenue à la température ambiante (20°C) et sous agitation, par exemple effectuée à l'aide d'un agitateur à hélice à 4 pales inclinées.

Un test similaire est conduit afin de déterminer la vitesse de dissolution des écailles dans l'acide acrylique.

Le test consiste à définir le temps nécessaire pour dissoudre la quantité d'écailles nécessaires pour obtenir une concentration finale d'hydroquinone de 2 % en masse dans l'acide acrylique.

Cette vitesse s'échelonne entre 30 min et 1 h selon l'épaisseur des écailles.

Mesurées dans les mêmes conditions, les vitesses de dissolution de la poudre d'hydroquinone dans l'eau et dans l'acide acrylique sont respectivement de 9 min et 20 min.

Il est à noter que les temps de dissolution de l'hydroquinone mise en forme selon l'invention sont légèrement accrus mais cette augmentation est acceptable par l'utilisateur compte tenu des avantages obtenus par ailleurs.

L'invention n'exclut pas suite à l'étape de mise en forme, une étape supplémentaire permettant de calibrer les écailles obtenues.

Ainsi, les écailles peuvent être introduites par exemple dans un granulateur à couteaux ou à barres permettant de réduire la taille des particules afin d'avoir une répartition plus homogène dans les trois dimensions et obtenir ainsi de l'hydroquinone sous forme de particules isotropes.

Par «particules isotropes », on entend des particules avec trois dimensions équivalentes.

Les particules obtenues s'approchent de la forme cubique et présentent un côté pouvant varier entre 400 et 1500 µm, de préférence entre 500 et 750 µm.

Ainsi, les écailles peuvent être utilisées comme produit intermédiaire pour fabriquer de l'hydroquinone sous forme de particules isotropes.

L'hydroquinone ainsi obtenue présente une densité accrue.

Selon un autre aspect plus spécifique, la présente invention a également pour objet un dispositif pour la mise en oeuvre du procédé de l'invention permettant d'obtenir des écailles d'hydroquinone purifiée.

Ce dispositif, qui se présente le plus souvent sous la forme d'une installation de dimensions industrielles, comprend :
- une première colonne de distillation adaptée à la distillation d'étêtage d'une hydroquinone brute *HQ⁰* selon l'étape (A) précitée, conçue pour éliminer le résorcinol en tête de colonne et récupérer en pied de colonne un mélange contenant la majeure partie de l'hydroquinone et les impuretés lourdes ; et
- une deuxième colonne de distillation adaptée à la distillation d'équeutage de l'étape (B) précitée, dont l'entrée est reliée au pied de colonne de la première colonne, conçue pour éliminer en pied de colonne le pyrogallol contenu dans le mélange brut provenant du pied de colonne de la première colonne de distillation, et pour obtenir en tête de colonne l'hydroquinone sous forme purifiée ;
- un dispositif permettant la mise en forme sous forme d'écailles de l'hydroquinone purifiée obtenue en sortie de distillation comprenant un support en un matériau ou revêtu d'un matériau conducteur de la chaleur qui peut être sous forme d'un cylindre en rotation associé à des moyens de refroidissement du support et de récupération des écailles d'hydroquinone.

Dans ce dispositif, les deux colonnes de distillation présentent avantageusement les caractéristiques préférentielles telles que définies plus haut dans la présente description. En particulier, les colonnes sont de préférence des colonnes à double enveloppe chauffées par un fluide caloporteur du type précité.

Pour ce qui est du dispositif de mise en forme, il est avantageusement placé dans une enceinte équipée de moyens permettant d'assurer une atmosphère inerte, de préférence l'azote.

L'invention sera encore explicitée davantage par le biais de la description qui suit, faite en référence aux figures ci-annexées, où :

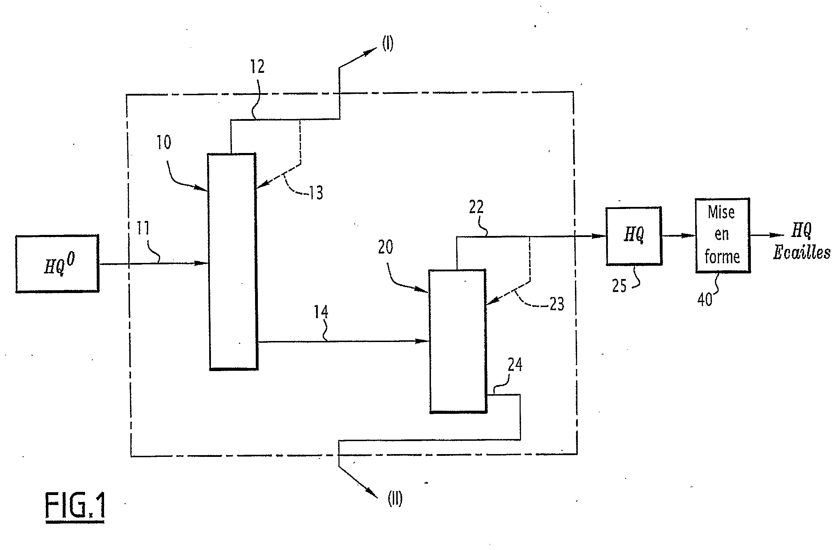

La Figure 1 est une représentation schématique du dispositif général utilisé selon l'invention pour la mise en oeuvre des étapes de distillation (A) et (B) et de mise en forme de l'hydroquinone issue de la distillation.

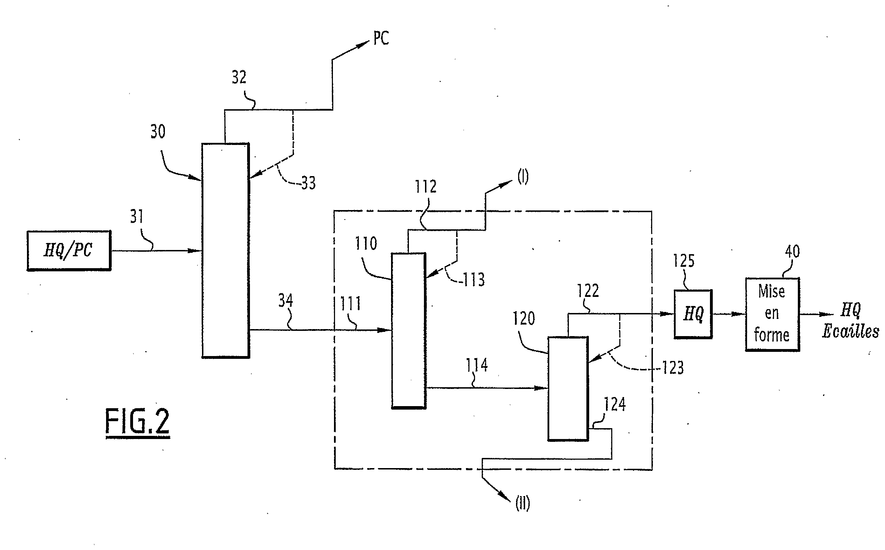

La Figure 2 est une représentation schématique d'un dispositif mettant en oeuvre une variante spécifique de l'invention, selon laquelle on prépare hydroquinone purifiée à partir d'un mélange tel qu'issu d'une réaction d'hydroxylation de phénol par du peroxyde d'hydrogène en présence d'un catalyseur de type acide protique fort.

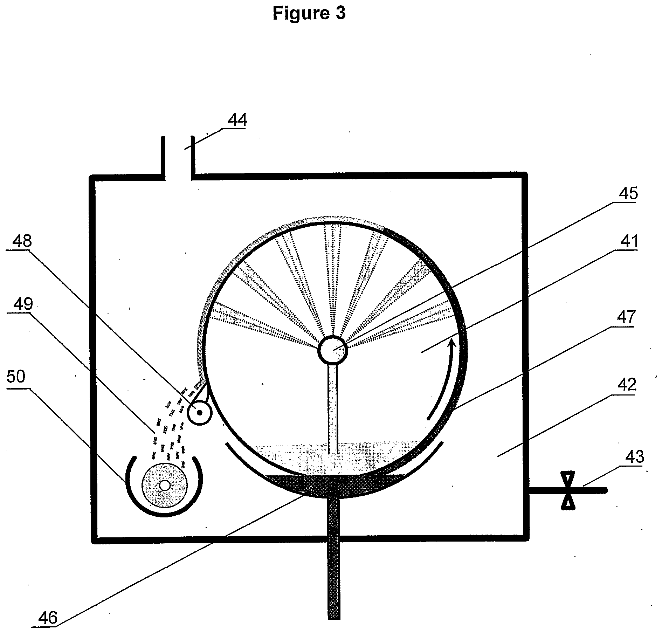

La Figure 3 est une représentation schématique d'un dispositif permettant la mise en forme de l'hydroquinone, sur un cylindre en rotation.

Sur la Figure 1, qui illustre le principe général du procédé de l'invention, on introduit l'hydroquinone brute *HQ*⁰ (11) qui est un mélange d'hydroquinone, d'impuretés légères contenant du résorcinol et d'impuretés lourdes contenant du pyrogallol, dans une première colonne de distillation (10) : l'alimentation de la colonne (10) est de préférence localisée sensiblement à mi-hauteur de la colonne.

La distillation d'étêtage [étape (A)] qui s'opère dans la colonne (10) conduit, au niveau de la tête de colonne (10), à l'élimination d'un flux symbolisé par (I) sur la figure 1, comprenant du résorcinol et éventuellement d'autres impuretés légères, ainsi qu'une fraction de l'hydroquinone. Ce flux (I) venant de la tête de colonne peut être ensuite traité, recyclé ou éliminé, notamment par un traitement dans un brûleur. Le flux (12) qui sort en tête de colonne (10) est dérivé en partie (13) pour être réinjecté dans la colonne (10), généralement latéralement en tête de colonne, pour assurer le reflux dans la colonne, reflux qui est mis en place notamment pour améliorer l'efficacité de la séparation.

Parallèlement, on obtient au niveau du pied de colonne (10), un mélange brut M (14) contenant de l'hydroquinone et d'impuretés lourdes, avec éventuellement des traces d'impuretés légères non extraites lors de l'étape précédente. Ce mélange brut M est introduit dans une deuxième colonne de distillation (20), l'alimentation de la colonne (20) étant de préférence sensiblement à mi-hauteur. Dans cette colonne (20), le mélange brut M issu de la colonne (10) va subir la distillation d'équeutage de l'étape (B).

Dans la colonne (20), la distillation conduit à la séparation d'une part, d'une hydroquinone purifiée HQ (22) récupérée en tête de colonne (20) et obtenue après condensation, sous forme liquide et d'autre part, d'un flux (24) symbolisé par (II) sur la figure 1, comprenant du pyrogallol et éventuellement d'autres impuretés lourdes (ainsi qu'un peu d'hydroquinone) qui sont évacués au niveau du pied de colonne (20). Ce flux (II) qui sort en pied de colonne (20) est ensuite traité, éliminé ou recyclé. Là encore, il est avantageux de mettre en place un reflux (23) en dérivant une partie du flux (22), notamment pour améliorer l'efficacité de la séparation des impuretés lourdes

On obtient en tête de la colonne (20), une hydroquinone (22) purifiée ne contenant essentiellement plus d'impuretés et qui a été liquéfiée après passage dans un condenseur.

Cette hydroquinone liquide et purifiée est ensuite stockée avant d'être mise en forme, dans un bac de stockage (25) maintenu sous atmosphère inerte et à l'état liquide.

Sur la Figure 2, un dispositif du type précité est mis en oeuvre dans un procédé plus complexe de traitement d'un mélange *HQ*/*PC* du type obtenu à l'issu d'une hydroxylation de phénol par le peroxyde d'hydrogène en présence d'un catalyseur, ce mélange *HQ*/*PC* comprenant essentiellement du pyrocatéchol (PC) et de l'hydroquinone, et de faible quantités d'impuretés légères (résorcinol) et d'impuretés lourdes (pyrogallol).

Ce mélange *HQ*/*PC* (31) alimente une première colonne de distillation (30) destinée à éliminer substantiellement le pyrocatéchol (32) en tête de colonne. Là encore, il est avantageux de mettre en place un reflux (33) en dérivant une partie du flux (32). On obtient en tête de colonne (30), un pyrocatéchol purifié (32).

Quel que soit le mode de mise en oeuvre de la distillation dans la colonne (30), on obtient en pied de colonne (30), une hydroquinone brute *HQ⁰* (34) contenant essentiellement de l'hydroquinone (typiquement entre 96 et 99,5 %), associée à de faibles quantités d'impuretés, à savoir de l'ordre de 0,1 à 2 % d'impuretés légères (résorcinol et traces de pyrocatéchol) et de l'ordre de 0,1 à 2 % d'impuretés lourdes (pyrogallol pour l'essentiel).

Cette hydroquinone brute *HQ⁰* est ensuite soumise à un traitement de distillation d'étêtage dans la colonne (110), où les impuretés légères (résorcinol et traces de pyrocatéchol) sont éliminées en tête de colonne (110), sous la forme d'un flux symbolisé par (I) sur la figure 2. Le flux qui sort en tête de colonne (112) est dérivé en partie (113) pour être réinjecté dans la colonne (110) pour assurer le reflux. Au niveau du pied de colonne (110), on récupère un mélange brut (114) contenant de l'hydroquinone et les impuretés lourdes (pyrogallol essentiellement).

Le mélange brut ainsi obtenu est introduit dans la colonne (120), de préférence sensiblement à mi-hauteur. Il subit dans cette colonne une distillation d'équeutage, conduisant à une récupération en tête de colonne (120), d'hydroquinone purifiée HQ (122), avantageusement munie d'un reflux (123). Les impuretés lourdes sont quant à elles, éliminées en pied de colonne (120) sous la forme d'un flux (124) symbolisé par (II) sur la figure 2.

On obtient ainsi à la sortie de la colonne (122) une hydroquinone à l'état liquide, purifiée qui est ensuite stockée dans un bac de stockage (125) maintenue sous atmosphère inerte et à l'état liquide.

Elle est ensuite convoyée vers un dispositif de mise en forme (40) tel que représenté par la Figure 3 permettant finalement de récupérer l'hydroquinone sous forme d'écailles.

Ainsi, le procédé illustré sur la Figure 2 permet la séparation efficace, sous forme isolée et purifiée (donc valorisable), des deux constituants principaux (pyrocatéchol et hydroquinone) contenus dans les milieux réactionnels issus d'une réaction d'hydroxylation de phénol par du peroxyde d'hydrogène en présence d'un catalyseur acide de type acide protique fort. Ce procédé peut être conduit selon un mode continu. Ce mode de réalisation particulier du procédé constitue un objet spécifique de la présente invention.

Sur la Figure 3, les écailles d'hydroquinone sont obtenues par solidification d'hydroquinone fondue sur un cylindre (41) en acier inoxydable (316) en rotation dans une enceinte (42) dans laquelle est établie une atmosphère appauvrie en oxygène par l'introduction d'azote (43). Le gaz chargé en vapeurs d'hydroquinone est évacué de l'enceinte en direction d'un dispositif de traitement des gaz (44).

La température du cylindre est régulée par l'intermédiaire d'une pulvérisation d'eau sur sa face interne (45). Il n'y a pas de contact direct entre l'eau de refroidissement et le produit.

L'hydroquinone à l'état liquide provenant du bac de stockage (25) ou (125) est introduite dans un bac d'alimentation (46) dont la température est régulée par une double enveloppe dans laquelle circule un fluide caloporteur. Le cylindre trempe dans l'hydroquinone en fusion et, du fait de sa rotation, entraîne à sa surface externe un film de produit fondu (47).

Au contact du métal froid, ce film de produit se solidifie progressivement pour arriver solidifier au niveau d'un couteau raclant (48) qui le décolle du cylindre sous forme d'écailles (49).

Les écailles ainsi obtenues sont collectées dans l'auge d'une vis de transport (50) qui les extrait de l'enceinte.

Le procédé de l'invention permet à partir d'une hydroquinone brute d'obtenir de manière simple et économique, de l'hydroquinone purifiée et facilement manipulable.

L'hydroquinone telle qu'obtenue selon l'invention présente par ailleurs, une très faible teneur en produits de dégradation thermique, telles que des quinones, en particulier lorsque le procédé est conduit en évitant tout contact de l'hydroquinone chauffée avec l'oxygène. Cette faible teneur en produit de dégradation se traduit par une absence sensible de coloration de l'hydroquinone purifiée obtenue, qui a un aspect blanc. La coloration de l'hydroquinone peut être mesurée plus précisément, par analyse colorimétrique d'une solution aqueuse de ladite hydroquinone, typiquement en réalisant une solution à 5 % en masse à température ambiante. On peut obtenir pour les hydroquinones purifiées de l'invention des indices de colorimétrie faibles, entre 20 et 200 Hazen et préférentiellement entre 20 et 100 Hazen.

On précise que les différentes pourcentages pondéraux donnés dans le présent texte, en rapport à l'hydroquinone sous forme solide sont exprimés par rapport à un produit sec obtenu après séchage jusqu'à obtention d'un poids constant.

On donne un exemple de réalisation de l'invention donné à titre illustratif et sans caractère limitatif.

### Exemple :

### 1. Purification de l'hydroquinone brute :

On a utilisé un dispositif tel que représenté sur la Figure 1, employé en continu, pour effectuer la purification d'une hydroquinone brute contenant, en masse par rapport à la masse totale de l'hydroquinone brute, 0,6 % de résorcinol et 0,7 % de pyrogallol.

Ce mélange a été introduit dans la première colonne de distillation (10) avec un débit d'alimentation constant de 100 kg/h.

La colonne (10) utilisée présente les caractéristiques suivantes :
- nombre d'étages théoriques : 30
- température en tête de colonne : 202°C
- pression de travail : 87 mBar
- taux de reflux: 600

Le temps de séjour de l'hydroquinone dans la colonne (10) est évalué à 25 min.

En tête de la colonne (10), on a obtenu un flux (I) comprenant du résorcinol, le résorcinol ayant un débit de 1 kg/h.

La deuxième colonne (20), dans laquelle le mélange brut obtenu en pied de la première colonne (10) a été introduit, a quant à elle, été utilisée dans les conditions suivantes :
- nombre d'étages théoriques : 30
- température en tête de colonne : 201°C
- pression de travail : 73 mBar
- taux de reflux : 7

Le temps de séjour de l'hydroquinone dans la colonne (20) est évalué à 30 min.

En pied de la colonne (20), le flux (24) comprenant le pyrogallol a un débit de 1 kg/h.

En tête de la colonne (20), on a obtenu une hydroquinone purifiée *HQ* sortant avec un débit de 98 kg/h.

Cette hydroquinone comprend moins de 2000 ppm de résorcinol et moins de 200 ppm de pyrogallol.

Elle a donc une pureté de 99,78 % en masse.

L'hydroquinone obtenue présente une coloration de 30 Hazen.

L'hydroquinone purifiée *HQ*, récupérée en tête de colonne (22) est stockée avant d'être mise en forme dans un bac de stockage (25) maintenu sous atmosphère d'azote et à une température comprise entre 178°C et 185°C assurée par circulation d'un fluide caloporteur dans la double enveloppe dont il est muni.

### 2. Mise en forme de l'hydroquinone purifiée:

L'étape de mise en forme de l'hydroquinone purifiée est mise en oeuvre dans l'appareillage décrit ci-dessous et schématisé par la figure 3.

Les écailles d'hydroquinone sont obtenues par solidification d'hydroquinone fondue sur un cylindre (41) en acier inoxydable (316) en rotation dans une enceinte (42) dans laquelle est établie une atmosphère appauvrie en oxygène par l'introduction d'azote (43). Le gaz chargé en vapeurs d'hydroquinone est évacué de l'enceinte en direction d'un dispositif de traitement des gaz (44).

La température du cylindre est régulée par l'intermédiaire d'une pulvérisation d'eau sur sa face interne (45). Il n'y a pas de contact direct entre l'eau de refroidissement et le produit.

L'hydroquinone à l'état liquide provenant du bac de stockage (25) représenté sur la Figure 1, est introduite dans un bac d'alimentation (46) dont la température est régulée par une double enveloppe dans laquelle circule un fluide caloporteur. Le cylindre trempe dans l'hydroquinone en fusion et, du fait de sa rotation, entraîne à sa surface externe un film de produit fondu (47).

Au contact du métal froid, ce film de produit se solidifie progressivement pour arriver solide au niveau d'un couteau raclant (48) qui le décolle du cylindre sous forme d'écailles (49).

Les écailles ainsi obtenues sont collectées dans l'auge d'une vis de transport (50) qui les extrait de l'enceinte.

La mise en forme de l'hydroquinone à l'état liquide est faite sur un cylindre qui a une surface de 0,75 m² (longueur = 0,48 m - diamètre = 0,50 m).

Les conditions de fonctionnement sont les suivantes :
- la vitesse de rotation du cylindre : V = 2 tours/min,
- la température de l'eau de refroidissement : Te = 60°C,
- la hauteur d'immersion du cylindre dans le produit en fusion : H = 25 mm,
- la température du produit fondu : Tp = 180 °C.

Des écailles sont obtenues d'une épaisseur de 0,95 mm avec une productivité de 98 kg/h.

Le % massique de particules passant à travers un tamis de maille de 100 µm est de 0,8 %.

La masse volumique apparente non tassée (g/cm³) est de 0,52.

La masse volumique apparente tassée (g/cm³) est de 0,70.

Le temps de dissolution dans l'eau à 20°C (obtention d'une solution à 4,8 % en masse) est de 21 minutes.

Le temps de dissolution dans l'acide acrylique à 20°C (obtention d'une solution à 2,0 % en masse) est de 45 minutes.

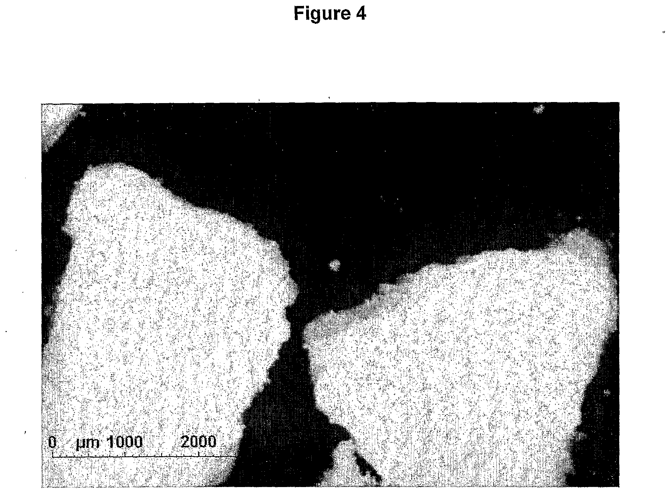

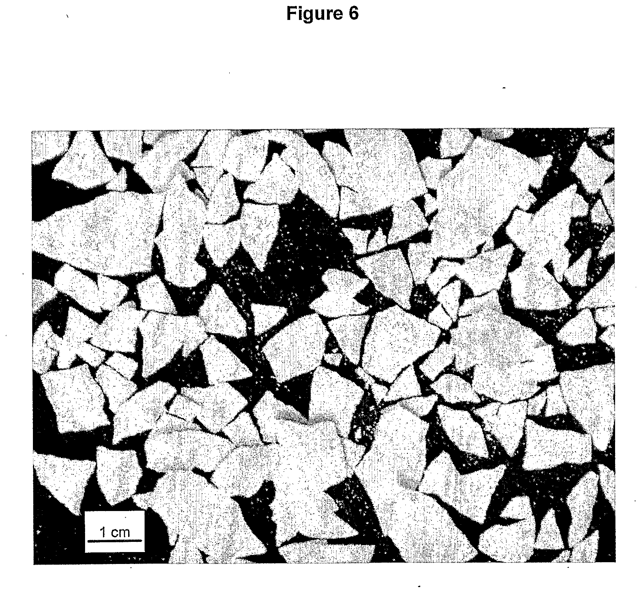

La photographie de la figure 4 illustre la morphologie du produit obtenu selon l'invention. Une vue d'ensemble du produit obtenu est donnée par la figure 6.

## Revendications

1. Procédé de préparation d'hydroquinone purifiée et mise en forme sous forme d'écailles dont le pourcentage en masse de particules de dimension inférieure à 100 µm est inférieur à 3%, à partir d'une hydroquinone brute *HO^{o}* contenant de 96 à 99,5 % en masse d'hydroquinone, et de 0,5 et 4 % en masse d'impuretés, lesdites d'impuretés incluant au moins du résorcinol, du pyrogallol et des traces de pyrocatéchol **caractérisé par le fait qu'**il comprend les étapes suivantes :
- une étape de purification de l'hydroquinone brute par distillation comprenant :
(A) une distillation d'étêtage, dans laquelle on injecte l'hydroquinone brute *HQ*⁰ dans une colonne de distillation et où on élimine le résorcinol en tête de distillation éventuellement conjointement à tout ou partie des autres impuretés légères ayant une température de vaporisation inférieure à celle de l'hydroquinone, ce par quoi on récupère en pied de colonne un mélange brut M comprenant de l'hydroquinone et les impuretés lourdes ; et
(B) une distillation d'équeutage dans laquelle on injecte le mélange brut M obtenu dans l'étape (A) dans une colonne de distillation et où on élimine le pyrogallol en pied de colonne éventuellement conjointement à tout ou partie des autres impuretés lourdes ayant une température de vaporisation supérieure à celle de l'hydroquinone, ce par quoi on récupère en tête de colonne de l'hydroquinone sous une forme purifiée *(HQ)* ;
les étapes de distillation (A) et (B) étant conduites sous atmosphère inerte et de telle sorte que la température dans les colonnes de distillation soit d'au moins 175°C, la pression soit comprise entre 50 et 100 millibars et le temps de séjour inférieur à 1 heure,
- une étape de mise en forme de l'hydroquinone purifiée obtenue en sortie de distillation, par dépôt de celle-ci, à l'état liquide, en film sur un support en un matériau ou revêtu d'un matériau conducteur de la chaleur suivie par sa solidification en amenant le support à la température adéquate, puis récupération de l'hydroquinone solidifiée sous forme d'écailles, à l'aide de tout moyen approprié.

2. Procédé selon la revendication 1, **caractérisé par le fait que** l'hydroquinone brute *HQ⁰* contient de 0,1 à 2 % en masse d'impuretés légères.

3. Procédé selon la revendication 1 ou la revendication 2, **caractérisé par le fait que** l'hydroquinone brute *HQ⁰* contient de 0,1 à 2 % en masse d'impuretés lourdes.

4. Procédé selon l'une des revendications 1 à 3, **caractérisé par le fait que** les impuretés légères présentes dans l'hydroquinone brute *HQ⁰* comprennent au moins 50 % de résorcinol, par rapport à la masse totale des impuretés légères.

5. Procédé selon l'une des revendications 1 à 4, **caractérisé par le fait que** les impuretés lourdes présentes dans l'hydroquinone brute *HQ⁰* comprennent au moins 50 % de pyrogallol, par rapport à la masse totale des impuretés lourdes.

6. Procédé selon l'une des revendications 1 à 5, **caractérisé par le fait que** l'hydroquinone brute *HQ⁰* est obtenue à partir d'un mélange réactionnel issu d'une hydroxylation de phénol par le peroxyde d'hydrogène en présence de catalyseurs acides, après élimination substantielle du pyrocatéchol par distillation.

7. Procédé selon l'une des revendications 1 à 6, **caractérisé par le fait que** l'hydroquinone brute *HQ⁰* comprend, en masse par rapport à la quantité totale d'hydroquinone brute :
- de 96 à 99,5 % d'hydroquinone,
- de 0,1 à 2 % de résorcinol,
- de 0,1 à 2 % pyrogallol
- éventuellement du pyrocatéchol sous forme de traces.

8. Procédé selon l'une des revendications 1 à 7, **caractérisé par le fait que**, dans l'étape (A), le point d'alimentation où est introduit l'hydroquinone brute *HQ⁰* est sensiblement à mi-hauteur de la colonne de distillation, avec un rapport volumique de la zone de rectification de la colonne de l'étape (A) à la zone d'épuisement de la colonne de l'étape (A) compris entre 25 : 75 et 75 : 25.

9. Procédé selon l'une des revendications 1 à 8, **caractérisé par le fait que** le flux qui sort en tête de la colonne de distillation de l'étape (A), est dérivé en partie, pour être réinjecté dans la colonne de distillation, avec un taux de reflux compris entre 300 et 2000.

10. Procédé selon l'une des revendications 1 à 9, **caractérisé par le fait que** le nombre d'étages théoriques de la colonne utilisée dans l'étape (A) est d'au moins 20.

11. Procédé selon l'une des revendications 1 à 10, **caractérisé par le fait que** le temps de séjour de l'hydroquinone dans la colonne de l'étape (A) est compris entre 10 minutes et 1 heure.

12. Procédé selon l'une des revendications 1 à 11, **caractérisé par le fait que**, dans l'étape (B), le point d'alimentation où est introduit le mélange brut M est sensiblement à mi-hauteur de la colonne de distillation, avec un rapport volumique de la zone de rectification de la colonne de l'étape (B) à la zone d'épuisement de la colonne de l'étape (B) compris entre 25 :75 et 75 :25.

13. Procédé selon l'une des revendications 1 à 12, **caractérisé par le fait que** le flux qui sort en tête de la colonne de distillation de l'étape (B), est dérivé en partie, pour être réinjecté dans la colonne de distillation, avec un taux de reflux compris entre 1 et 15.

14. Procédé selon l'une des revendications 1 à 13, **caractérisé par le fait que** le nombre d'étages théoriques de la colonne utilisée dans l'étape (B) est d'au moins 20.

15. Procédé selon l'une des revendications 1 à 14, **caractérisé par le fait que** le temps de séjour de l'hydroquinone dans la colonne de l'étape (B) est compris entre 10 minutes et 1 heure.

16. Procédé selon l'une des revendications 1 à 15, **caractérisé par le fait que** les étapes (A) et (B) sont conduites à des pressions comprises entre 60 et 90 millibars.

17. Procédé selon la revendication 1 **caractérisé par le fait que** l'on établit dans l'enceinte où l'hydroquinone est mise en forme, une atmosphère de gaz inertes.

18. Procédé selon l'une des revendications 1 et 17 **caractérisé par le fait que** le matériau sur lequel est déposé l'hydroquinone a une conductibilité thermique d'au moins 10 W/m.K.

19. Procédé selon la revendication 18 **caractérisé par le fait que** le support est ou est revêtu d'un acier inoxydable.

20. Procédé selon la revendication 1_ ou la revendication 19 **caractérisé par le fait que** l'hydroquinone à l'état liquide est déposée sur une surface refroidie consistant ou revêtu d'un matériau conducteur qui peut être sous forme d'une bande transporteuse, d'un ou de plateau(x) tournant(s) ou bien d'un cylindre en rotation.

21. Procédé selon la revendication 20 **caractérisé par le fait que** le cylindre est placé dans 1 à 10 cm d'hydroquinone fondue placée dans une auge d'alimentation, le dépôt sur le cylindre s'effectue par trempage.

22. Procédé selon la revendication 20 **caractérisé par le fait que** l'alimentation de l'hydroquinone à l'état liquide peut se faire sur le cylindre par l'intermédiaire d'un rouleau applicateur, lui-même alimenté en hydroquinone fondue ou par coulée par gravité ou par l'intermédiaire d'une pompe.

23. Procédé selon l'une des revendications 20 à 22 **caractérisé par le fait que** la vitesse de rotation du cylindre varie entre 0,5 et 20 tours/min.

24. Procédé selon l'une des revendications 20 à 23 **caractérisé par le fait que** le cylindre est maintenu une température comprise entre 20°C et 80°C.

25. Procédé selon l'une des revendications 20 à 24 **caractérisé par le fait que** le cylindre est refroidi par circulation d'eau dans une double enveloppe ou par pulvérisation d'eau à l'intérieur du cylindre.

26. Procédé selon l'une des revendications 20 à 25 **caractérisé par le fait qu'**il comprend les étapes suivantes :
- débarrasser l'oxygène de l'enceinte dans laquelle est effectuée l'opération de mise en forme,
- déposer l'hydroquinone à l'état liquide en film sur un cylindre maintenu à une température comprise entre 20°C et 80°C,
- maintenir l'hydroquinone suffisamment longtemps sur le cylindre afin qu'elle se solidifie.
- récupérer le produit solidifié à l'aide de tout moyen approprié.

27. Procédé selon l'une des revendications 1 à 26 **caractérisé par le fait que** l'on récupère l'hydroquinone mise en forme à l'aide d'un couteau qui racle le cylindre et décolle la couche de produit qui est récupéré.

28. Procédé selon l'une des revendications 1 à 27 **caractérisé par le fait que** l'on soumet les écailles obtenues à une opération de calibrage permettant d'obtenir une répartition plus homogène de la taille des particules.

29. Procédé selon la revendication 28 **caractérisé par le fait que** l'opération est effectuée dans un granulateur à couteaux ou à barres.

## Patentansprüche

1. Verfahren zur Herstellung von Hydrochinon, das eine Aufreinigung und Formgebung erfahren hat, wobei es in Form von Schuppen vorliegt, deren Massenprozentanteil an Partikeln, welche eine Abmessung von weniger als 100 µm aufweisen, niedriger als 3 % ist, ausgehend von einem rohen Hydrochinon *HQ⁰,* welches 96 bis 99,5 Massen-% an Hydrochinon und 0,5 bis 4 Massen-% an Verunreinigungen enthält, wobei diese Verunreinigungen mindestens Resorcin, Pyrogallol und Spuren von Brenzcatechin beinhalten, **dadurch gekennzeichnet, dass** es die folgenden Schritte umfasst:
- einen Schritt der Aufreinigung des rohen Hydrochinons durch Destillation, welcher Folgendes umfasst:
(A) eine Destillation zur Entfernung von Kopfprodukten, wobei das rohe Hydrochinon *HQ⁰* in eine Destillationskolonne eingeleitet wird und wobei das Resorcin kopfseitig der Destillation entfernt wird, möglicherweise gemeinsam mit einem Teil oder der Gesamtheit der weiteren leichtflüchtigen Verunreinigungen mit einer Verdampfungstemperatur unterhalb derjenigen des Hydrochinon, sodass sumpfseitig der Kolonne eine Rohmischung M entnommen wird, die das Hydrochinon und die schwerflüchtigen Verunreinigungen umfasst; und
(B) eine Destillation zur Entfernung von Sumpfprodukten, wobei die Rohmischung M, welche im Schritt (A) erhalten wurden, in eine Destillationskolonne eingeleitet wird und wobei das Pyrogallol sumpfseitig der Kolonne entfernt wird, möglicherweise gemeinsam mit einem Teil oder der Gesamtheit der weiteren schwerflüchtigen Verunreinigungen mit einer Verdampfungstemperatur, die oberhalb derjenigen der Hydrochinons liegt, sodass kopfseitig der Kolonne das Hydrochinon in aufgereinigter Form *(HQ)* entnommen wird;
wobei die Destillationsschritte (A) und (B) unter Inertgasatmosphäre durchgeführt werden und zwar derart, dass die Temperatur in den Destillationskolonnen mindestens 175 °C beträgt, der Druck im Bereich von 50 bis 100 Millibar liegt und die Verweildauer weniger als eine Stunde beträgt,
- einen Schritt der Formgebung des aufgereinigten Hydrochinons, wie es ausgangsseitig der Destillation erhalten wird, indem dieses, in flüssiger Form, als Dünnschicht auf einen Träger aufgebracht wird, der aus einem Material besteht oder mit einem Material beschichtet ist, welches wärmeleitend ist, um es anschließend zum Erstarren zu bringen, indem der Träger auf die angemessene Temperatur gebracht wird, woraufhin das erstarrte Hydrochinon mit Hilfe eines beliebigen zweckmäßigen Mittels in Form von Schuppen gewonnen wird.

2. Verfahren nach Anspruch 1, **dadurch gekennzeichnet, dass** das rohe Hydrochinon *HQ⁰* 0,1 bis 2 Massen-% an leichtflüchtigen Verunreinigungen enthält.

3. Verfahren nach Anspruch 1 oder Anspruch 2, **dadurch gekennzeichnet, dass** das rohe Hydrochinon *HQ⁰* 0,1 bis 2 Massen-% an schwerflüchtigen Verunreinigungen enthält.

4. Verfahren nach einem der Ansprüche 1 bis 3, **dadurch gekennzeichnet, dass** die leichtflüchtigen Verunreinigungen, welche in dem rohen Hydrochinon *HQ⁰* vorliegen, mindestens 50 % an Resorcin umfassen, bezogen auf die Gesamtmasse der leichtflüchtigen Verunreinigungen.

5. Verfahren nach einem der Ansprüche 1 bis 4, **dadurch gekennzeichnet, dass** die schwerflüchtigen Verunreinigungen, welche in dem rohen Hydrochinon *HQ⁰* vorliegen, mindestens 50 % an Pyrogallol umfassen, bezogen auf die Gesamtmasse der schwerflüchtigen Verunreinigungen.

6. Verfahren nach einem der Ansprüche 1 bis 5, **dadurch gekennzeichnet, dass** das rohe Hydrochinon *HQ⁰* ausgehend von einer Reaktionsmischung erhalten wird, die aus einer Hydroxylierung von Phenol mittels Wasserstoffperoxid in Gegenwart von sauren Katalysatoren stammt, nachdem das Brenzcatechin im Wesentlichen durch Destillation entfernt wurde.

7. Verfahren nach einem der Ansprüche 1 bis 6, **dadurch gekennzeichnet, dass** das rohe Hydrochinon *HQ⁰* Folgendes umfasst, nach Masse unter Bezugnahme auf die Gesamtmenge an rohem Hydrochinon:
- 96 bis 99,5 % an Hydrochinon,
- 0,1 bis 2 % an Resorcin,
- 0,1 bis 2 % an Pyrogallol
- möglicherweise Brenzcatechin in Form von Spuren.

8. Verfahren nach einem der Ansprüche 1 bis 7, **dadurch gekennzeichnet, dass** in Schritt (A) die Einspeisestelle, an welcher das rohe Hydrochinon *HQ⁰* eingeleitet wird, sich im Wesentlichen auf halber Höhe der Destillationskolonne befindet, wobei das Volumenverhältnis zwischen dem Rektifizierungsabschnitt der Kolonne des Schrittes (A) und dem Abtriebsabschnitt der Kolonne des Schrittes (A) im Bereich von 25:75 bis 75:25 liegt.

9. Verfahren nach einem der Ansprüche 1 bis 8, **dadurch gekennzeichnet, dass** der kopfseitige Ausgangsstoffstrom der Destillationskolonne des Schrittes (A) teilweise abgeführt wird, um wieder in die Destillationskolonne eingeleitet zu werden, mit einem Rücklaufverhältnis im Bereich von 300 bis 2.000.

10. Verfahren nach einem der Ansprüche 1 bis 9, **dadurch gekennzeichnet, dass** die Anzahl an theoretischen Böden der Kolonne, welche im Schritt (A) verwendet wird, mindestens 20 beträgt.

11. Verfahren nach einem der Ansprüche 1 und 10, **dadurch gekennzeichnet, dass** die Verweildauer des Hydrochinons in der Kolonne des Schrittes (A) im Bereich von 10 Minuten bis einer Stunde liegt.

12. Verfahren nach einem der Ansprüche 1 bis 11, **dadurch gekennzeichnet, dass** in Schritt (B) die Einspeisestelle, an welcher die Rohmischung M eingeleitet wird, sich im Wesentlichen auf halber Höhe der Destillationskolonne befindet, wobei das Volumenverhältnis zwischen dem Rektifizierungsabschnitt der Kolonne des Schrittes (B) und dem Abtriebsabschnitt der Kolonne des Schrittes (B) im Bereich von 25:75 bis 75:25 liegt.

13. Verfahren nach einem der Ansprüche 1 bis 12, **dadurch gekennzeichnet, dass** der kopfseitige Ausgangsstoffstrom der Destillationskolonne des Schrittes (B) teilweise abgeführt wird, um wieder in die Destillationskolonne eingeleitet zu werden, mit einem Rücklaufverhältnis im Bereich von 1 bis 15.

14. Verfahren nach einem der Ansprüche 1 bis 13, **dadurch gekennzeichnet, dass** die Anzahl an theoretischen Böden der Kolonne, welche im Schritt (B) verwendet wird, mindestens 20 beträgt.

15. Verfahren nach einem der Ansprüche 1 und 14, **dadurch gekennzeichnet, dass** die Verweildauer des Hydrochinons in der Kolonne des Schrittes (B) im Bereich von 10 Minuten bis einer Stunde liegt.

16. Verfahren nach einem der Ansprüche 1 bis 15, **dadurch gekennzeichnet, dass** die Schritte (A) und (B) bei Drücken im Bereich von 60 bis 90 Millibar durchgeführt werden.

17. Verfahren nach Anspruch 1, **dadurch gekennzeichnet, dass** in dem geschlossenen Raum, in welchem das Hydrochinon seine Formgebung erfährt, eine Atmosphäre aus Inertgasen hergestellt wird.

18. Verfahren nach einem der Ansprüche 1 bis 17, **dadurch gekennzeichnet, dass** das Material, auf welches das Hydrochinon aufgebracht wird, eine Wärmeleitfähigkeit von mindestens 10 W/m.K aufweist.

19. Verfahren nach Anspruch 18, **dadurch gekennzeichnet, dass** der Träger einem nichtrostenden Stahl ist entspricht oder mit einem solchen beschichtet ist.

20. Verfahren nach Anspruch 1 oder Anspruch 19, **dadurch gekennzeichnet, dass** das Hydrochinon in flüssigem Zustand auf eine gekühlte Oberfläche aufgebracht wird, die aus einem leitfähigen Material besteht oder damit beschichtet ist, wobei es in Form eines Förderbandes oder eines/mehrerer Drehteller(s) oder auch eines rotierenden Zylinders vorliegen kann.

21. Verfahren nach Anspruch 20, **dadurch gekennzeichnet, dass** der Zylinder in 1 bis 10 cm an geschmolzenem Hydrochinon gegeben wird, welches in einen Speisetrog gegeben wurde, wobei das Auftragen auf den Zylinder durch Eintauchen erfolgt.

22. Verfahren nach Anspruch 20, **dadurch gekennzeichnet, dass** das Einspeisen des Hydrochinons im flüssigen Zustand derart erfolgen kann, dass es mit Hilfe einer Auftragerolle auf den Zylinder gelangt, welcher wiederum geschmolzenes Hydrochinon zugeführt wird, oder dass es schwerkraftgetrieben oder mit Hilfe einer Pumpe gegossen wird.

23. Verfahren nach einem der Ansprüche 20 bis 22, **dadurch gekennzeichnet, dass** die Rotationsgeschwindigkeit des Zylinders zwischen 0,5 und 20 Umdrehungen/Min. variiert.

24. Verfahren nach einem der Ansprüche 20 bis 23, **dadurch gekennzeichnet, dass** der Zylinder bei einer Temperatur im Bereich von 20 °C und 80 °C gehalten wird.

25. Verfahren nach einem der Ansprüche 20 bis 24, **dadurch gekennzeichnet, dass** der Zylinder durch Umwälzen von Wasser in einem Doppelmantel oder durch Versprühen von Wasser im Inneren des Zylinders gekühlt wird.

26. Verfahren nach einem der Ansprüche 20 bis 25, **dadurch gekennzeichnet, dass** es die folgenden Schritte umfasst:
- Entfernen des Sauerstoffs aus dem geschlossenen Raum, in welchem der Vorgang der Formgebung durchgeführt wird,
- Aufbringen des Hydrochinons in flüssigem Zustand als Dünnschicht auf einen Zylinder, der bei einer Temperatur im Bereich von 20 °C bis 80 °C gehalten wird,
- Belassen des Hydrochinons auf dem Zylinder über einen Zeitraum, der dafür ausreicht, dass es erstarrt,
- Gewinnen des erstarrten Produkts mit Hilfe eines beliebigen geeigneten Mittels.

27. Verfahren nach einem der Ansprüche 1 bis 26, **dadurch gekennzeichnet, dass** das Hydrochinon, nachdem es die Formgebung erfahren hat, mit Hilfe eines Messers gewonnen wird, welches den Zylinder abschabt und die Schicht aus Produkt abhebt, sodass dieses gewonnen wird.

28. Verfahren nach einem der Ansprüche 1 bis 27, **dadurch gekennzeichnet, dass** die erhaltenen Schuppen einem Größensortierungsvorgang unterzogen werden, der es ermöglicht, eine homogenere Verteilung der Partikelgröße zu erzielen.

29. Verfahren nach Anspruch 28, **dadurch gekennzeichnet, dass** der Vorgang in einem Granulator mit Messern oder mit Stäben erfolgt.

## Claims

1. Process for the preparation of purified hydroquinone put into the form of flakes, the percentage by weight of particles with dimensions of less than 100µm of which is less than 3%, starting from a crude hydroquinone *HQ⁰* comprising from 96 to 99.5% by weight of hydroquinone and from 0.5 to 4% by weight of impurities, said impurities including at least resorcinol, pyrogallol and traces of pyrocatechol, **characterized in that** it comprises the following stages:
- a stage of purification of the crude hydroquinone by distillation, comprising:
(A) a topping distillation, in which the crude hydroquinone *HQ⁰* is injected into a distillation column and where the resorcinol is removed as distillation top product, optionally in conjunction with all or part of the other light impurities having a lower evaporation temperature than that of hydroquinone, whereby a crude mixture M, comprising hydroquinone and the heavy impurities, is recovered at the column bottom; and
(B) a tailing distillation, in which the crude mixture M obtained in stage (A) is injected into a distillation column and where the pyrogallol is removed at the column bottom, optionally in conjunction with all or part of the other heavy impurities having a higher evaporation temperature than that of hydroquinone, whereby hydroquinone in a purified form (*HQ*) is recovered at the column top;
the distillation stages (A) and (B) being carried out under an inert atmosphere and so that the temperature in the distillation columns is at least 175°C, the pressure is between 50 and 100 millibar and the residence time is less than 1 hour,
- a stage of forming the purified hydroquinone obtained at the distillation outlet, by deposition of the latter in the liquid state as a film on a support made of a thermally conductive material or coated with a thermally conductive material, followed by the solidification thereof by bringing the support to the appropriate temperature, then recovery of the solidified hydroquinone in the form of flakes, using any appropriate means.

2. Process according to Claim 1, **characterized in that** the crude hydroquinone *HQ⁰* comprises from 0.1 to 2% by weight of light impurities.

3. Process according to Claim 1 or Claim 2, **characterized in that** the crude hydroquinone *HQ⁰* comprises from 0.1 to 2% by weight of heavy impurities.

4. Process according to one of Claims 1 to 3, **characterized in that** the light impurities present in the crude hydroquinone *HQ⁰* comprise at least 50% of resorcinol, with respect to the total weight of the light impurities.

5. Process according to one of Claims 1 to 4, **characterized in that** the heavy impurities present in the crude hydroquinone *HQ⁰* comprise at least 50% of pyrogallol, with respect to the total weight of the heavy impurities.

6. Process according to one of Claims 1 to 5, **characterized in that** the crude hydroquinone *HQ⁰* is obtained from a reaction mixture resulting from a hydroxylation of phenol by hydrogen peroxide in the presence of acid catalysts, after substantial removal of the pyrocatechol by distillation.

7. Process according to one of Claims 1 to 6, **characterized in that** the crude hydroquinone *HQ⁰* comprises, by weight with respect to the total amount of crude hydroquinone:
- from 96 to 99.5% of hydroquinone,
- from 0.1 to 2% of resorcinol,
- from 0.1 to 2% of pyrogallol,
- optionally pyrocatechol in the form of traces.

8. Process according to one of Claims 1 to 7, **characterized in that**, in stage (A), the feed point where the crude hydroquinone *HQ⁰* is introduced is substantially at mid-height in the distillation column, with a ratio by volume of the rectification region of the column of stage (A) to the stripping region of the column of stage (A) of between 25:75 and 75:25.

9. Process according to one of Claims 1 to 8, **characterized in that** the stream which exits at the top of the distillation column of stage (A) is partially diverted, in order to be reinjected into the distillation column, with a reflux ratio of between 300 and 2000.

10. Process according to one of Claims 1 to 9, **characterized in that** the number of theoretical stages of the column used in stage (A) is at least 20.

11. Process according to one of Claims 1 to 10, **characterized in that** the residence time of the hydroquinone in the column of stage (A) is between 10 minutes and 1 hour.

12. Process according to one of Claims 1 to 11, **characterized in that**, in stage (B), the feed point where the crude mixture M is introduced is substantially at mid-height in the distillation column, with a ratio by volume of the rectification region of the column of stage (B) to the stripping region of the column of stage (B) of between 25:75 and 75:25.

13. Process according to one of Claims 1 to 12, **characterized in that** the stream which exists at the top of the distillation column of stage (B) is partially diverted, in order to be reinjected into the distillation column, with a reflux ratio of between 1 and 15.

14. Process according to one of Claims 1 to 13, **characterized in that** the number of theoretical stages of the column used in stage (B) is at least 20.

15. Process according to one of Claims 1 to 14, **characterized in that** the residence time of the hydroquinone in the column of stage (B) is between 10 minutes and 1 hour.

16. Process according to one of Claims 1 to 15, **characterized in that** stages (A) and (B) are carried out at pressures of between 60 and 90 millibar.

17. Process according to Claim 1, **characterized in that** an atmosphere of inert gases is established in the chamber where the hydroquinone is formed.

18. Process according to either of Claims 1 and 17, **characterized in that** the material on which the hydroquinone is deposited has a thermal conductivity of at least 10 W/m.K.

19. Process according to Claim 18, **characterized in that** the support is or is coated with a stainless steel.

20. Process according to Claim 1 or Claim 19, **characterized in that** the hydroquinone in the liquid state is deposited on a cold surface consisting of or coated with a conductive material which can be in the form of a conveyor belt, of one or more turntable (s) or else of a rotating cylinder.

21. Process according to Claim 20, **characterized in that** the cylinder is placed in 1 to 10 cm of molten hydroquinone placed in a feed trough and deposition on the cylinder takes place by dipping.

22. Process according to Claim 20, **characterized in that** the feeding of the hydroquinone in the liquid state can be carried out on the cylinder via an applicator roll, itself fed with molten hydroquinone, or by pouring by gravity or via a pump.

23. Process according to one of Claims 20 to 22, **characterized in that** the rotational speed of the cylinder varies between 0.5 and 20 revolutions/min.

24. Process according to one of Claims 20 to 23, **characterized in that** the cylinder is maintained at a temperature of between 20°C and 80°C.

25. Process according to one of Claims 20 to 24, **characterized in that** the cylinder is cooled by circulation of water in a jacket or by spraying water inside the cylinder.

26. Process according to one of Claims 20 to 25, **characterized in that** it comprises the following stages:
- freeing from oxygen the chamber in which the forming operation is carried out,
- depositing the hydroquinone in the liquid state as a film on a cylinder maintained at a temperature of between 20°C and 80°C,
- maintaining the hydroquinone on the cylinder for a sufficient length of time for it to solidify,
- recovering the solidified product using any appropriate means.

27. Process according to one of Claims 1 to 26, **characterized in that** the hydroquinone formed is recovered using a blade which scrapes the cylinder and detaches the layer of product, which is recovered.

28. Process according to one of Claims 1 to 27, **characterized in that** the flakes obtained are subjected to a grading operation which makes it possible to obtain a more homogeneous distribution in the size of the particles.

29. Process according to claim 28, **characterized in that** the operation is carried out in a blade or bar granulator.
